(19) Europäisches Patentamt
European Patent Office
Office européen des brevets

(11) Veröffentlichungsnummer: **0 087 585**
**B1**

# (12) EUROPÄISCHE PATENTSCHRIFT

(45) Veröffentlichungstag der Patentschrift: **06.11.85**

(51) Int. Cl.⁴: **C 07 C 120/00,** C 07 C 121/34

(21) Anmeldenummer: **83100738.0**

(22) Anmeldetag: **27.01.83**

## (54) Verfahren zur Herstellung von 3-Alkoxi-acrylnitrilen.

(30) Priorität: **26.02.82 DE 3206878**

(43) Veröffentlichungstag der Anmeldung: **07.09.83 Patentblatt 83/36**

(45) Bekanntmachung des Hinweises auf die Patenterteilung: **06.11.85 Patentblatt 85/45**

(84) Benannte Vertragsstaaten: **BE CH DE FR GB IT LI NL**

(56) Entgegenhaltungen: **EP-A-0 018 473** **GB-A-2 010 271**

(73) Patentinhaber: **DYNAMIT NOBEL AKTIENGESELLSCHAFT, Postfach 1209, D-5210 Troisdorf, Bez. Köln (DE)**

(72) Erfinder: **Theis, Christoph, Dr., Breniger Strasse 11, D-5303 Bornheim 4 (DE)**
Erfinder: **Prange, Uwe, Dr., Porzer Strasse 7, D-5216 Niederkassel 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 087 585 B1

**Beschreibung**

Die Erfindung betrifft ein Verfahren zur Herstellung von 3-Alkoxi-acrylnitrilen durch Umsetzung eines ggf. substituierten aliphatischen Nitrils mit Kohlenmonoxid und Metallalkoholaten bei erhöhtem Druck und Temperatur und nachfolgender Alkylierung mit einem organischen Halogenid.

Nach dem Stand der Technik können α-Formyl-Alkali bzw. Erdalkalisalze nach der DE-OS Nr. 2753322 aus den Nitrilen hergestellt werden. Diese können in einer gesonderten Reaktion nach der EP-PS Nr. 0018473 bzw. der DE-OS Nr. 2912345 3-Alkoxiacrylnitrile durch Umsetzung mit Halogeniden der Formel $R^3$-Hal bilden. Die Ausführung beider Reaktionen nacheinander mit im Regelfall verschiedenen Lösungsmitteln und Hilfsstoffen ist umständlich und führt zu Stoffverlusten.

Der vorliegenden Erfindung liegt daher die Aufgabe zugrunde, 3-Alkoxi-acrylnitrile durch ein einfach ausführbares Verfahren herzustellen, wobei Hilfsstoffe und Lösungsmittel eingespart und eine vereinfachte Aufarbeitung möglich werden soll.

Die Aufgabe wird erfindungsgemäss dadurch gelöst, dass die ggf. substituierten aliphatischen Nitrile der Formel I mit Kohlenmonoxid und Alkoholat zur Reaktion gebracht werden und ohne Isolierung der entstehenden α-Formyl-Alkali- bzw. Erdalkalisalze der Nitrile, mit Stoffen der Formel $R^3$ Hal zum Produkt umgesetzt werden, wobei die Nitrile zugleich als Ausgangsstoff und als Lösungsmittel dienen.

Gegenstand der Erfindung ist daher das Verfahren nach Anspruch 1.

Durch den Überschuss des Nitrils werden die Alkoholate praktisch vollständig verbraucht, wodurch gegenüber dem Stand der Technik die notwendige Menge der Alkoholate vermindert ist, und nicht mehr die andernfalls gesonderte Aufarbeitung der Alkali- bzw. Erdalkalisalze der α-Hydroxymethylennitrile behindert.

Diese Verfahrensweise ermöglicht einen sehr niedrigen Verbrauch von Kohlenmonoxid. Bei der Aufarbeitung des Produkts ist es dadurch möglich, durch eine Destillation ohne Fraktionierung ein Stoffgemisch aus Ammeisensäureester, Alkohol, Resten des Halogenids $R^3$-Hal und dem überschüssigen Nitril einfach abzutrennen, das wieder verwendbar ist.

Als Alkoholate der Formel III können Alkali- oder Erdalkalialkoholate eingesetzt werden. Bevorzugt sind Na- und K-Salze des Methanols oder Ethanols wegen ihrer leichten Zugänglichkeit.

Sehr bevorzugt wird die Reaktion in Gegenwart einer als Stabilisator wirkenden basisch reagierenden Verbindung der Alkali oder Erdalkalimetalle duchgeführt. Beispielsweise können Alkali- oder Erdalkalihydroxide, -hydrogencarbonate, -carbonate oder Erdalkalioxide, vorzugsweise Ca $(OH)_2$ eingesetzt werden. Die basisch reagierende Verbindung wird in einem Verhältnis von 0,05 bis 1, vorzugsweise 0,4 bis 0,6 Äquivalent je Mol eingesetztes Alkoholat, zugegeben. Sehr bevorzugt werden weiterhin zur Erhöhung von Selektivität und Ausbeute als Katalysatoren tertiäre Amine oder quaternäre Ammoniumbasen verwendet. Die tert. Amine können gleiche oder ggf. verschiedene, lineare oder verzweigte Cycloalkyl-, Aralkyl- oder Alkyl-Reste mit 1 bis 20 C-Atomen enthalten. Von den cyclischen Resten sind monocyclische Reste und von den aliphatischen Resten die mit 1 bis 6 C-Atomen bevorzugt.

Die quaternären Ammoniumsalze können die genannten Reste oder Aryl- bzw. Alkarylreste und ein einwertiges Anion enthalten. Von den cyclischen Resten sind moncyclische Reste, von den aliphatischen Resten die mit 1 bis 6 C-Atomen bevorzugt. Bevorzugte Katalysatoren sind Trimethylamin, Triethylamin und Tetra-n-butylammoniumchlorid bzw. -bromid. Die Katalysatoren werden in Konzentrationen von 1 bis 10 Gew.-%, vorzugsweise 3 bis 5 Gew.-%, bezogen auf die eingesetzte Alkoholatmenge, zugesetzt.

Als Halogenide der Formel IV werden die Chlorverbindungen bevorzugt. Die ebenfalls einsetzbaren Bromide oder Jodide bringen gegenüber den Chlorverbindungen keine Vorteile.

Das Kohlenmonoxid kann im Druckbereich von 5 bis 100, vorzugsweise 10 bis 50 bar, angewendet werden. Höhere Drucke sind möglich, aber nicht notwendig. Das CO kann mit Fremdgasen wie Stickstoff oder Wasserstoff gemischt sein. Die Reaktion mit den Halogeniden erfolgt zwischen Normaldruck und 50 bar, wobei Normaldruck oder geringer Überdruck bevorzugt ist. Die Temperatur kann zwischen 20 und 220° C liegen. Bei der Umsetzung mit Kohlenmonoxid sind Temperaturen von 35 bis 100° C bevorzugt. Bei der Umsetzung mit den Halogeniden liegen die bevorzugten Temperaturen im Bereich von 90 bis 150° C.

Da die als Ausgangsstoffe verwendeten Nitrile gleichzeitig Lösungsmittel sind, wird deren Menge so bemessen, dass das Reaktionsgemisch gut rührbar ist. Im allgemeinen werden 5 bis 20 Mol Nitril je Äquivalent des Alkoholats verwendet. Dadurch wird das Alkoholat praktisch vollständig umgesetzt. Durch den Überschuss des Nitrils wird überraschend die Bildung von Ameisensäureester aus dem frei werdenden Alkohol und Kohlenmonoxid weitgehend unterdrückt. Der Verbrauch von Kohlenmonoxid liegt bei nur 1,2 bis 1,7 Mol CO je Mol Alkoholat, wobei zudem der Ameisensäureester erneut als CO-Quelle einsetzbar ist.

Die Halogenide der Formel II werden in Mengen von 1 bis 2 Mol je Äquivalent des Alkoholats eingesetzt. Der Überschuss der Halogenide kann zurückgewonnen werden.

Das Verfahren ergibt insgesamt, besonders unter Berücksichtigung der wiederverwendbaren Ausgangsstoffe und Hilfsstoffe, eine beträchtliche Einsparung von Stoffen, Energie und Zeitaufwand. Alle Ausgangs- und Hilfsstoffe, mit Ausnahme der Halogenide, können bereits am Beginn der Reaktion zugesetzt werden.

Es ist vorteilhaft, dass Jodide bei der Reaktion nicht erforderlich sind. Die Mengen der genannten basisch reagierenden Verbindungen sind verhältnismässig klein. In vorteilhafter Weise entstehen nur 4 bis 6 Gew.-% C-alkylierter Produkte.

Die Reaktionszeiten sind mit 1 bis 2 Stunden kurz, jedoch ist nach Zugabe der Halogenide eine Nachreaktionszeit zur Vervollständigung der Umsetzung zweckmässig. Nach Ende der Reaktionszeit werden feste Bestandteile durch Filtration oder Zentrifugieren entfernt. In sehr vorteilhafter Weise können durch Destillation zunächst die Leichtsieder und darauf das Produkt von den Feststoffen getrennt werden.

Das Reaktionsprodukt besteht aus einem E-/Z-Isomerengemisch. Bei Einsatz von Nitrilen, in denen $R^1 = H$ ist, enthält das β-Alkoxinitril kleine Mengen des durch Addition des Reaktionsalkohols $R^2OH$ gebildeten Acetals sowie einer Verbindung, die durch Alkylierung in 2-Stellung den Rest $R^3$ enthält.

Das Acetal kann bei der Aufarbeitung durch Zusatz von Mineralsäuren wie $H_3PO_4$, $H_2SO_4$ oder HCl oder saurer Salze von Mineralsäuren, wie z.B. $KHSO_4$, zum gewünschten β-Alkoxi-acrylinitril gespalten werden. Dadurch wird gleichzeitig das jeweilige Z-Isomere in das stabilere E-Isomere umgelagert. Das Alkylierungsprodukt wird durch fraktionierte Destillation abgetrennt. Zweckmässig sollen die Reste $R^2$ und $R^3$ übereinstimmen.

*Beispiel 1*

In ein Gemisch aus 1100 g Acetonitril, 102,0 g (1,5 Mol) Natriumethoxid, 55,5 g (0,75 Mol) $Ca(OH)_2$ und 5,1 g Triethylamin werden in einem 2-l-Autoklaven mit Hubrühr-Einrichtung bei 40 bar CO-Druck und 60° C Kohlenmonoxid eingeleitet, bis nach 30 Minuten kein weiteres CO aufgenommen wurde. Danach werden bei Normaldruck 193,5 g (3,0 Mol) Ethylchlorid zugegeben und die Suspension 6 Stunden bei 120° C gerührt. Darauf wird durch Filtration der Feststoff entfernt, mit Acetonitril gewaschen und die vereinigten Filtrate fraktioniert destilliert. Bei Normaldruck wird ein Gemisch aus Ameisensäureester, Alkohol, Acetonitril und Resten des Ethylchlorids abdestilliert. Der Rückstand wird im Vakuum fraktioniert destilliert. Als Produkt werden 119,6 g Destillat vom Kp = 80 bis 92° C (13 Torr) erhalten. Gaschromatographische Zusammensetzung: 82,3 Fl.-% (Z/E)-3-Ethoxi-propennitril (A), 9,0 Fl.-% 3,3-Diethoxi-propannitril (B) und 8,7 Fl.-% (Z/E)-3-Ethoxi-2-ethyl-propennitril (C). Die Gesamtmenge an (A) und (B), berechnet als (A), ergibt eine Ausbeute von 73,4%, bezogen auf das Alkoholat.

*Beispiel 2*

Entsprechend Beispiel 1, jedoch mit 1000 g Acetonitril, erfolgt die Umsetzung mit Kohlenmonoxid. Nach Zugabe des Ethylchlorids bei 120° C wird die erhaltene Suspension unter guter mechanischer Rührung destilliert.

Man erhält 122 g Destillat:
(A) = 57,8 Fl.-%
(B) = 31,9 Fl.-%
(C) = 7,8 Fl.-%

Ausbeute von (A) aus (A) und (B): 70,1%, bezogen auf das Alkoholat.

*Beispiel 3*

Entsprechend Beispiel 1, jedoch unter Verwendung von 2,9 g Tetra-n-butylammonium-chlorid, anstelle von Triethylamin, werden 119,0 g Destillat (83,2 Fl.-% (A), 8,5 Fl.-% (B) und 8,3 Fl.-%) erhalten. Ausbeute: 72,8%, bezogen auf das Alkoholat.

*Beispiel 4*

Entsprechend Beispiel 1, jedoch unter Verwendung von 145,1 g (2,25 Mol) Ethylchlorid, werden als Produkt 104,3 g Destillat (74,1 Fl.-% (A), 16,7 Fl.-% (B) und 9,2 Fl.-% (C)) erhalten. Die Ausbeute, berechnet als (A), beträgt 61,2%, bezogen auf das Alkoholat.

*Beispiel 5*

Entsprechend der Verfahrensweise des Beispiels 1, jedoch unter Verwendung von 81,0 g (1,5 Mol) Natriummethoxid, wird bei 70° C und 50 bar CO das α-Formyl-Natriumsalz hergestellt und mit 151,5 g (3,0 Mol) Methylchlorid wie in Beispiel 1 umgesetzt. Bei der destillativen Aufarbeitung erhält man 89,5 g Destillat vom Kp = 64 bis 81° C / 12 Torr (gaschromatographisch; 78,5 Fl.-% (Z/E)-3-Methoxi-propennitril, 17,6 Fl.-% 3,3-Dimethoxi-propannitril und 3,7 Fl.-% (Z/E)-3-Methoxi-2-methyl-propennitril). Ausbeute: 66,0%, berechnet als 3-Methoxi-propionitril, bezogen auf das Alkoholat.

*Beispiel 6*

Beispiel 1 wird wiederholt, jedoch werden 1100 g Propionitril, anstelle von Acetonitril, bei 75° C und 50 bar CO umgesetzt. Die Vakuum-Destillation ergibt 121,4 g Destillat vom Kp = 81 bis 99° C / 12 Torr (93,2 Fl.-% (Z/E)-3-Ethoxi-2-methyl-propennitril und 6,6 Fl.-% 3,3-Diethoxi-2-methyl-propannitril). Ausbeute: 71,3%, bezogen auf das Alkoholat.

*Beispiel 7*

Entsprechend Beispiel 6 werden 1000 g Propionitril mit 81,0 g (1,5 Mol) Natriummethoxid bei 80° C mit CO von 50 bar Druck umgesetzt und mit 151,5 g (3,0 Mol) Methylchlorid zur Reaktion gebracht. Die Vakuum-Destillation ergibt 100,8 g (Kp = 68 bis 73 / 12 Torr) Destillat. Ausbeute: 69,3% (Z/E)-3-Methoxi-2-methyl-propennitril, bezogen auf das Alkoholat.

*Beispiel 8*

Ein Reaktionsgemisch wie in Beispiel 1 mit Gehalten von 1000 g Butyronitril wird bei 85° C mit Kohlenmonoxid von 45 bar Druck zur Reaktion gebracht und entsprechend Beispiel 1 mit 193,5 g (1,5 Mol) Ethylchlorid umgesetzt. Produkt: 122,0 g Destillat (Kp = 87 bis 106 / 12 Torr) Ausbeute: 65,0%, berechnet als 3-Ethoxi-2-ethyl-propennitril.

**Patentansprüche**

1. Verfahren zur Herstellung von 3-Alkoxi-acrylnitrilen der Formel

$$R^3O-CH=C(R^1)-CN \quad (I)$$

worin $R^1$ die Bedeutung H, geradkettige oder verzweigte Alkylreste mit 1 bis 20 C-Atomen, geradkettige oder verzweigte Reste $-(CH_2)_n-CN$, $-(CH_2)_nOR^4$ oder $-(CH_2)_n-CHOR^4)_2$ mit der Bedeutung n = 0 bis 5 und Cyc = isocyclische oder heterocyclische, ein- oder mehrkernige aromatische oder cycloaliphatische Ringsysteme, welche gegebenenfalls Substituenten an den Ringen tragen, hat; und worin $R^3$ die Bedeutung geradkettige oder verzweigte Alkyl- oder Alkenylreste mit 1 bis 12 C-Atomen, isocyclische oder heterocyclische, ein- oder mehrkernige aromatische oder cycloaliphatische Ringsysteme, welche gegebenenfalls Substituenten tragen, oder $-(CH_2)_p-Cyc$ mit Cyc in der vorherigen Bedeutung, die Reste $-(CH_2)_p-OR^4$ oder $-(CH_2-CH_2)_q-R^4$ mit p = 1 bis 5 und q = 1 bis 4 sowie $R^4$ entsprechend der vorherigen Bedeutung hat; dadurch gekennzeichnet, dass eine Verbindung der Formel

$$R^1-CH_2-CN \quad (II)$$

worin $R^1$ die genannte Bedeutung hat, mit einem Alkoholat der Formel

$$M(OR^2)m \quad (III)$$

worin M ein Alkalimetall mit m = 1 oder ein Erdalkalimetall mit m = 2 und $R^2$ ein geradkettiger oder verzweigter Alkylrest mit 1 bis 5 C-Atomen ist, mit Kohlenmonoxid bei erhöhtem Druck und Temperatur in das entsprechende α-Formyl-Alkali- oder Erdalkali-Salz der Verbindung II übergeführt und dieses Salz ohne weitere Behandlung der entstandenen Reaktionsmischung mit einer Halogenverbindung der Formel

$$R^3\text{-Hal} \quad (IV)$$

worin $R^3$ die genannte Bedeutung hat und Hal Chlor, Brom oder Jod bedeutet, bei erhöhter Temperatur umgesetzt wird, wobei das Nitril der Formel I als Ausgangsstoff und Lösungsmittel dient.

2. Verfahren nach Anspruch 1, dadurch gekennzeichnet, dass die Reaktionen in Gegenwart einer als Stabilisator wirkenden, basisch reagierenden Verbindung der Alkali- oder Erdalkalimetalle durchgeführt werden.

3. Verfahren nach einem der Ansprüche 1 oder 2, dadurch gekennzeichnet, dass als Katalysatoren tert. Amine oder quaternäre Ammoniumsalze verwendet werden.

4. Verfahren nach Anspruch 3, dadurch gekennzeichnet, dass der Katalysator in Mengen von 1 bis 10 Gew.-%, bezogen auf die eingesetzte Alkoholat-Menge, verwendet wird.

5. Verfahren nach mindestens einem der Ansprüche 1 bis 4, dadurch gekennzeichnet, dass die Reaktionstemperatur zwischen 20 und 220° C liegen.

6. Verfahren nach mindestens einem der Ansprüche 1 bis 5, dadurch gekennzeichnet, dass die Reaktionstemperatur der Umsetzung mit den Stoffen der Formel $R^3$-Hal zwischen 60 und 220° C liegt.

**Claims**

1. Process for the production of 3-alkoxy-acrylo-nitriles of the formula

$$R^3O-CH=C(R^1)-CN \quad (I)$$

wherein $R^1$ has the meaning H, straight-chained or branched-chained alkyl residues of 1-20 C-atoms, straight-chained or branched residues $-(CH_2)_n-CN$, $-(CH_2)_nOR^4$ or $-(CH_2)_n-CH(OR^4)_2$ with the meaning n = 0 to 5 and Cyc = isocyclic or heterocyclic mono- or polynuclear aromatic or cycloaliphatic ring systems, which optionally carry ring substituents; and wherein $R^3$ has the meaning straight-chained or branched alkyl or alkenyl residues with 1 to 12 C-atoms, isocyclic or heterocyclic mono- or polynuclear aromatic or cycloaliphatic ring systems, which optionally carry substituents, or $-(CH_2)_p-Cyc$ with Cyc having the foregoing meaning, the residues $-(CH_2)_p-OR^4$ or $-(CH_2-CH_2)_q-R^4$ with p = 1 to 5 and q = 1 to 4, as well as $R^4$ corresponding to the foregoing meaning; characterized in that a compound of the formula

$$R^1-CH_2-CN \quad (II)$$

wherein $R^1$ has the indicated meaning is converted with an alcoholate of the formula

$$M(OR^2)_m \quad (III)$$

wherein M is an alkali metal with m = 1 or an alkaline earth metal with m = 2 and $R^2$ is a straight-chained or branched chain alkyl residue with 1 to 5 C-atoms, with carbon monoxide at elevated pressure and temperature into the corresponding α-formyl-alkali or alkaline earth salt of the compound II and this salt is reacted, without further treatment of the existing reaction mixture, with a halogen compound of the formula

$$R^3\text{-Hal} \quad (IV)$$

wherein $R^3$ has the indicated meaning and Hal denotes chorine, bromine or iodine, at elevated temperature, with the nitrile of Formula I serving as starting material and solvent.

2. Process according to Claim 1, characterized in that the reactions are carried out in the presence

of a basically reacting compound of the alkali or alkaline earth metal acting as stabiliser.

3. Process according to one of Claims 1 or 2, characterized in that tertiary amines or quaternary ammonium salts are used as catalysts.

4. Process according to Claim 3, characterized in that the catalyst is used in amounts of 1 to 10% by weight, related to alcoholate amount used.

5. Process according to at least one of Claims 1 to 4, characterized in that the reaction temperature lies between 20 and 220° C.

6. Process according to at least one of Claims 1 to 5, characterized in that the reaction temperature of the reaction with the compounds of formula $R^3$-Hal lies between 60 and 220° C.

## Revendications

1. Procédé de préparation de 3-alcoxy-acrylonitriles de formule

$$R^3O-CH=\underset{\displaystyle R^1}{\underset{|}{C}}-CN \qquad (I)$$

dans laquelle $R^1$ désigne un atome de H, des radicaux alkyle linéaires ou ramifiés ayant 1 à 20 atomes de carbone, des radicaux linéaires ou ramifiés $-(CH_2)_nCN$, $-(CH_2)_nOR^4$ ou $-(CH_2)_n-CH(OR^4)_2$, n étant un nombre de 0 à 5 et cyc = des systèmes cycliques aromatiques ou cycloaliphatiques mono- ou polycycliques, isocycliques ou hétérocycliques, qui portent éventuellement des substituants sur les cycles; et dans laquelle $R^3$ désigne des radicaux alkyle ou alcényle linéaires ou ramifiés ayant 1 à 12 atomes de carbone, des systèmes cycliques aromatiques ou cycloaliphatiques mono- ou polycycliques, isocycliques ou hétérocycliques, qui portent éventuellement des substituants, ou bien $-(CH_2)_p-Cyc$, Cyc ayant la signification ci-dessus, les radicaux $-(CH_2)_p-OR^4$ ou $-(CH_2-CH_2)_q-R^4$, p étant un nombre de 1 à 5 et q étant un nombre de 1 à 4 et $R^4$ ayant la signification ci-dessus; procédé caractérisé en ce que l'on fait réagir un composé de formule

$$R^1-CH_2-CN \qquad (II)$$

dans laquelle $R^1$ a la signification indiquée, avec un alcoolate de formule

$$M(OR^2)m \qquad (III)$$

dans laquelle M est un métal alcalin avec m = 1 ou un métal alcalino-terreux avec m = 2 et $R^2$ est un radical alkyle linéaire ou ramifié ayant 1 à 5 atomes de carbone, de l'oxyde de carbone sous pression et à température élevées pour le transformer en le sel correspondant de métal alcalin ou alcalino-terreux du dérivé $\alpha$-formylé du composé II et en ce que l'on fait réagir ce sel, sans autre traitement du mélange réactionnel formé, avec un composé halogéné de formule

$$R^3\text{-Hal} \qquad (IV)$$

dans laquelle $R^3$ a la signification indiquée et Hal désigne le chlore, le brome ou l'iode, à température élevée, le nitrile de formule I servant de matière première et de solvant.

2. Procédé selon la revendication 1, caractérisé en ce que l'on réalise les réactions en présence d'un composé de métal alcalin ou alcalino-terreux à réaction basique, agissant comme stabilisant.

3. Procédé selon la revendication 1 ou 2, caractérisé en ce que l'on emploie comme catalyseurs des amines tertiaires ou des sels d'ammonium quaternaires.

4. Procédé selon la revendication 3, caractérisé en ce que l'on emploie le catalyseur en quantité de 1 à 10% en poids par rapport à la quantité d'alcoolate mise en jeu.

5. Procédé selon l'une quelconque des revendications 1 à 4, caractérisé en ce que la température de réaction est comprise entre 20 et 220° C.

6. Procédé selon l'une quelconque des revendications 1 à 5, caractérisé en ce que la température de réaction avec les substances de formule $R^3$-Hal est comprise entre 60 et 220° C.